Europäisches Patentamt

(19) European Patent Office

Office européen des brevets

(11) EP 0 885 587 A1

(12) EUROPEAN PATENT APPLICATION

(43) Date of publication:
23.12.1998 Bulletin 1998/52

(51) Int. Cl.$^6$: A61B 5/00

(21) Application number: 98304752.3

(22) Date of filing: 17.06.1998

(84) Designated Contracting States:
AT BE CH CY DE DK ES FI FR GB GR IE IT LI LU
MC NL PT SE
Designated Extension States:
AL LT LV MK RO SI

(30) Priority: 17.06.1997 GB 9712504

(71) Applicant: Harrison, David Keith
Durham, DH1 5AP (GB)

(72) Inventor: Harrison, David Keith
Durham, DH1 5AP (GB)

(74) Representative:
Murnane, Graham John et al
Murgitroyd & Company,
373 Scotland Street
Glasgow G5 8QA (GB)

(54) Thermal imaging method and apparatus

(57) An apparatus for dynamic imaging of the blood perfusion in skin measures blood flow in human or animal skin non-invasively, by means of processing skin thermographic data. An area of skin is first cooled, the cooling means is removed, and then, using an infra-red detector, thermographic data corresponding to skin temperature is obtained at a number of points on the area of skin over a period of time. For each point an exponential fit equation for the variation with time of the thermographic data is calculated. The coefficients of the exponential fit equation are displayed in graphical map form, and the map can be interpreted to provide information about the blood perfusion rate in the skin.

FIG 12

**Description**

This invention relates to an apparatus and method for dynamic imaging of blood perfusion in human or animal skin, in particular by means of measuring and processing skin thermographic data.

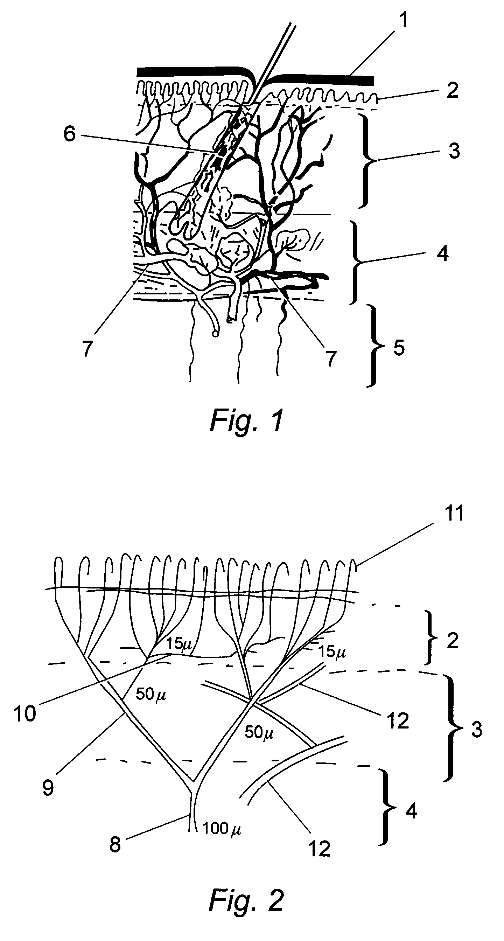

Many changes occur to the structure of human skin during growth. However, ignoring variation due to development and ageing, regional variations or the effects of pathology, there is a general pattern and structure of the vessels of the skin which is characteristic of most skin, for most of the time. This general pattern is presented in Fig. 1, which shows schematically the distribution of arteries and veins in a section of skin.

The *epidermis* (epithelial layer) 1 forms the outer waterproof boundary of the body and comprises a thin avascular layer less than 0.17 mm thick in most parts. Below this lies the highly vascular *dermis* 2, 3, 4 containing many blood and lymphatic vessels, where specialised structures (sweat glands, hair follicles 6, sensing organs) are located. Its thickness varies from 0.5 mm up to 4.0 mm.

The dermis can be subdivided into the papillary layer or upper dermis 2, the reticular layer or mid-dermis 3 and the deep dermis 4. The papillary layer 2 lies close to the epidermis 1 and contains a vascular complex, the sub-papillary plexus, which nourishes the base of the epidermis. Under this is the reticular dermis 3, containing a network of fibres and blood vessels that connect the papillary dermis 2 and the deep dermis 4.

The *deep dermis* 4 contains the sweat glands, the hair follicles 6 and most importantly, the major vascular network 7 supplying the whole dermis. Below the dermis there is a layer 5 consisting of adipose tissue or subcutaneous fat, which is not highly vascular and whose thickness varies greatly between individuals. An important characteristic of this layer is that it is a poorer conductor of heat than other body tissues. Therefore, it acts as an insulator reducing the total heat lost by the body.

The configuration of the vessels in the layers of the skin is shown in Fig. 2. An artery B about 100 μm in diameter enters the lower dermis 4. It has a lining of flat endothelial cells which lie on a relatively thick elastic membrane (internal elastic lamina). Around it there are several layers of smooth muscle, and collagen fibres.

The arteries divide once or twice as they pass through the deep dermis 4 and when their branches 9 reach the mid dermis 3 they divide again. Here they are about 50 μm in diameter and have only a single layer of smooth muscle cells. The smooth muscle layer becomes discontinuous, oblique and spiral and the vessels are now arterioles. No internal elastic lamina exists in the arterioles, and very few elastic fibres are detectable in the network supplying the muscle layer.

Further on, the repeated subdivision of the arterioles reduces their diameter down to approximately 10-15 μm forming the pattern of the capillaries 10 in the upper dermis (papillary dermis) 2. This is a network of horizontal capillaries lying parallel to the surface (sub-papillary plexus). Above it are the terminal capillaries 11. These are vertical loops which arise from the arterioles and drain it into the horizontal sub-papillary venus plexus.

The venules 12 in the upper and middle dermis are more numerous than the arterioles. Their diameter ranges from 40-60 μm in the upper and mid-dermis to 100-400 μm in the deeper tissues.

Some 80% or more of total body heat transfer occurs through the skin. The remaining part takes place in mucous membranes. Heat can be lost from the skin by four different means, namely radiation, conduction, con vention and evaporation.

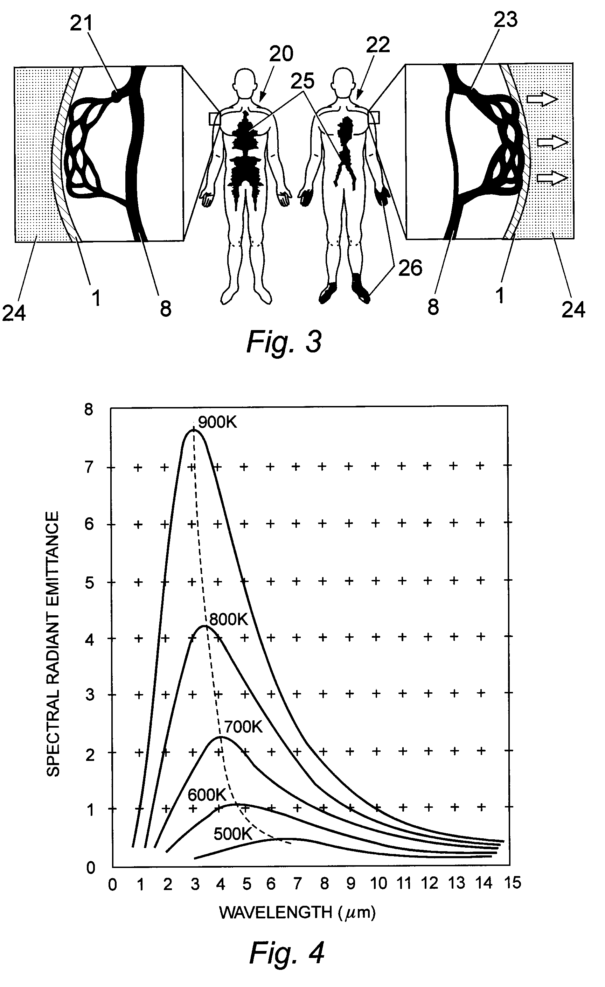

The basic mechanism of heat exchange in the human body through skin to the outside environment 24 can be seen in Fig. 3. When homeostasis requires that the body 20 conserves heat, blood flow in the skin decreases by vasoconstriction 21 to limit heat loss. When heat must be lost from the body 22 to maintain the stability of the internal environment, flow of warm blood to the skin increases by vasodilation 23.

The temperature of the skin surface is lower than that of the body core 25 because there is a constant heat loss from the body surface to the environment.

Conduction from the underlying tissues provides continuous, relatively small input of heat to the skin, but the main mechanism of heat exchange to the skin is by perfusion of blood, at core body temperature, from the main circulation. Additionally, for reasons associated with the basic thermal physics, heat transfer from the warm arterial blood to the surrounding tissues 26 occurs mainly in vessels greater than 50 μm in diameter.

For this reason skin temperature, especially at high perfusion rates, reflects heat exchange at the deeper levels of the dermis. Consequently an area of the skin with relatively high blood perfusion rate has a surface temperature higher than that of an area with lower perfusion.

Many areas of clinical medicine would benefit from a method of quantifying regional perfusion to the skin, for example, plastic surgery, amputation surgery, and cancer diagnosis. Different techniques have been developed through the years for this purpose, but most skin blood flow measurement techniques suffer from a number of limitations.

*Radioisotope clearance* involves measurement by a radiation detector of the rate of removal of an intradermally injected or transcutaneously absorbed amount of radioisotope. Flow can be quantified by this method but measurements are affected by needle trauma or by fat solubility problems in the case of Xenon[133].

Optical methods such as *Laser Doppler Flowmetry* and *Photoplethysmography*, although very popular because of their minimal interference with the skin, have some considerable disadvantages too.

Laser Doppler Flowmetry uses the Doppler shift of light reflected from a small volume of illuminated skin tissue to indicate the flux of moving red cells and therefore dermal perfusion in that portion of the skin. It has, though, uncertain penetration depth, measures in very small volumes of tissue, has a poor response to movement artefact, and gives only relative values of flow. Instrumentation applying this technique has been developed, using a scanning mirror rather than fibre optics in order to guide the laser beam. Using a scanning mirror may solve the spatial heterogeneity problem but it is still under trial and the produced measurements are only qualitative and slow to obtain.

Photoplethysmography uses visible or infra-red radiation reflected from the skin tissue to assess the blood flow in the skin layers. Although this technique indicates the timing of events, it provides a poor measure of change in volume, and it is very sensitive to motion artefact.

The *thermal clearance* method relates artificially induced temperature gradients longitudinal to a small region of the skin to blood flow in that region. This is also a point measurement technique which involves changes in dermal perfusion.

Medical thermography is a diagnostic method of imaging the distribution of temperature of human skin. It has a number of distinct advantages. It is completely non-invasive, a real time procedure, and there is no radiation hazard. This method can form the basis for regional mapping of skin blood flow, relating skin temperature with perfusion. However, static thermographic imaging is very dependent on environmental changes of temperature, the thermoregulatory status of the subject, and mechanisms of heat exchange that are not related to blood perfusion.

It is an object of the present invention to provide an apparatus and method for dynamic imaging of the blood perfusion rate in skin, which overcomes the problems of the prior art described above.

According to a first aspect of the present invention there is provided a method of measuring blood perfusion in human or animal skin, comprising the steps of:

(a) applying a cooling means to an area of skin;
(b) removing the cooling means from the area of skin;
(c) using an infra-red detector to obtain a first set of thermographic data $TD_1$ corresponding to skin temperature at a plurality of points on the area of skin at time $t_1$;
(d) repeating step (c) at least once to obtain a total of n sets of thermographic data $TD_1$ to $TD_n$ corresponding to times $t_1$ to $t_n$, where n is at least 2;
(e) calculating the coefficients of an exponential fit equation for the variation with time of the thermographic data $TD_1$ to $TD_n$ at each of the plurality of points; and
(f) displaying the coefficients of the exponential fit equation in graphical form.

Preferably the coefficients of the exponential fit equation are calculated using a curve stripping technique. Preferably n is at least 4, most preferably at least 10.

Preferably the coefficients of the exponential fit equation are displayed using colour mapping.

Preferably the cooling means is a cold object which s placed on the skin.

Preferably the method further includes the step of placing a reference object on the area of skin so that the thermographic data $TD_n$ is obtained at the same points on the area of skin at each successive repeat of step (c). The reference object may be a reflective strip.

According to a second aspect of the present invention there is provided a method of processing and displaying data relating to blood perfusion in human or animal skin, comprising the steps of:

(a) generating a series of n thermographic image matrices $IM_1$ to $IM_n$ corresponding to times $t_1$ to $t_n$, each thermographic image matrix comprising temperature data relating to a plurality of points on an area of skin;
(b) creating a filter matrix corresponding to a region of interest selected from the area of skin;
(c) applying the filter matrix to each thermographic image matrix $IM_1$ to $IM_n$ to obtain a cropped thermographic image matrix $IMCR_1$ to $IMCR_n$ having the value zero for points outside the region of interest;
(d) constructing an array $AR_m$ for each point m in the region of interest, the array containing temperature data $T_m$ at that point corresponding to times $t_1$ to $t_n$;
(e) for each array $AR_m$ performing a regression analysis to calculate the coefficients of the exponential components of the best fit equation

$$T_m = T_{min} + Ae^{at} + Be^{bt} + Ce^{ct}$$

where A, a, B, b, C, c are coefficients and $T_{min}$ is the minimum temperature data in each array;

(f) constructing a coefficient matrix ($Z_A$, $Z_a$ etc.) having as elements a parameter derived from the coefficients (A, a etc.) calculated in step (e) for each point m in the region of interest; and

(g) displaying the coefficient matrix as an image.

Preferably the parameter in step (f) is equal to one of the coefficients (A, a etc.) calculated in step (e). Alternatively the parameter in step (f) may be equal to the sum of the coefficients A, B and C calculated in step (e), representing the total tissue volume. Alternatively the parameter in step (f) may be equal to the sum of the coefficient products (aA + bB + cC) divided by the sum of the coefficients (A + B + C), representing the total flow.

Preferably the region of interest corresponds to an area of skin which has previously been cooled.

Preferably the coefficients of the exponential components are calculated using a curve stripping technique.

Preferably the minimum temperature data $T_{min}$ in each array is subtracted from each temperature data in the array before calculation of the coefficients.

Preferably the data corresponding to a first interval of time after cooling is discarded before calculating coefficients. The interval of time is preferably less than 10 seconds, more preferably less than 5 seconds, most preferably 3 or 4 seconds.

According to a third aspect of the present invention there is provided an apparatus for measuring blood perfusion in human or animal skin, comprising:

(a) an infra-red detecting means adapted to obtain periodically a set of thermographic data ($TD_1$ to $TD_n$) corresponding to skin temperature at a plurality of points on an area of skin;

(b) data storage means adapted to store each set of thermographic data ($TD_1$ to $TD_n$);

(c) computational means for calculating the coefficients of an exponential fit equation for the variation with time of the thermographic data ($TD_1$ to $TD_n$) at each of the plurality of points; and

(d) image display means adapted to display the coefficients of the exponential fit equation in graphical form.

The method of the present invention makes use of thermographic measurements (images) of *dynamic* changes in skin temperature, i.e. changes that are a result of the reheating of the skin after contact between the skin and a cold object.

The acquired images may be processed by suitable software. The purpose of this process is to perform a multi-exponential regression analysis to data that form reheat curves. The resultant coefficients of the components may then be reconstructed as new images.

The imaging techniques of the present invention may be applied to breast tumours, critical limb ischaemia and diabetic ulcers.

An embodiment of the invention will now be described with reference to the accompanying figures where:

Fig. 1 is a schematic drawing of the distribution of arteries and veins in the skin;

Fig. 2 shows the most common vascular pattern and structure in adult skin;

Fig. 3 is a simplified diagram showing the skin as a thermo-regulatory organ;

Fig. 4 shows black body spectral radiant emittance (Planck's Law);

Fig. 5 illustrates Planck's Law as a semi-log plot, showing a curve family from 100K to 1000 K;

Fig. 6 illustrates the operating principles of a pyroelectric detector;

Fig. 7 is a diagram to illustrate the concept of hot "core";

Figs. 8 to 10 show examples of Curve Stripping (step I, A component, step II, B component and step III, C component respectively);

Fig. 11 is a typical screen image showing the reheat data curve at a chosen location and the coefficients calculated by the software curve stripping method for the reheat data curve;

Fig. 12 shows a control thermographic image of an area on a forearm for normal skin data;

Figs. 13 to 15 shows the images produced by the method of the invention for the coefficients A and a; B and b; and C and c respectively for normal skin data;

Fig. 16 shows a control thermographic image of an area on a forearm for tuberculin reaction skin data; and

Figs. 17 to 19 shows the images produced by the method of the invention for the coefficients A and a; B and b; and C and c respectively for tuberculin reaction skin data.

The invention makes use of infra-red thermography, a known technique used to monitor the temperature distribution of a material by detecting the radiation that this material emits. The electromagnetic spectrum is divided into a number of wavelength regions, called "bands", distinguished by the methods used to produce or detect the radiation. The only difference between the bands is in wavelength. All the physical laws that govern these different regions are exactly the same. The background theory to the method of the invention is described below.

The *infra-red* band lies between 0.75 µm (the end of visual band) to 100 µm (the beginning of the microwave band).

It is subdivided into four sections, namely near infra-red (0.75 - 3 $\mu$m), middle infra-red (3-6 $\mu$m), far infra-red (6-15 $\mu$m) and extreme infra-red (15-100 $\mu$m).

Electromagnetic radiation is emitted spontaneously by all objects having a temperature above absolute zero (-273°C or 0K).

A blackbody is defined as an object which absorbs all incident radiation, and emits all its thermal energy in the form of electromagnetic radiation, at any wavelength. In other words a blackbody is a theoretical concept, that behaves as a perfect radiation absorber and emitter.

Human skin, at 37°C, has an emissivity of 0.98, and for this reason is a structure very close to a blackbody.

The spectral distribution of the radiation from a blackbody is described by means of the following formula:

$$W\lambda_b = \frac{2\pi xhc^2}{\lambda^5(e^{hc/\lambda kT}-1)} \times 10^{-6} \; [\text{Watts/m}^2 \; \mu\text{m}]$$

where:

$W\lambda_b =$ the blackbody spectral radiant emittance at wavelength $\lambda$
$c =$ the velocity of light ($3 \times 10^{-8}$ m/sec)
$h =$ Planck's constant ($6.6 \times 10^{-34}$ Joule sec)
$k =$ Boltzmann's constant ($1.4 \times 10^{-23}$ Joule/K)
$T =$ the absolute temperature of the blackbody (K)
$\lambda =$ wavelength (m)

This formula describes the relationship between the radiation energy and wavelength that is emitted by a blackbody in a certain temperature. Plank's formula, when plotted graphically for various temperatures, produces a family of curves. Following any particular Planck curve the spectral emittance is zero at $\lambda = 0$, then increases rapidly to a maximum at a wavelength $\lambda_{max}$, and then approaches zero again at very long wavelengths. The higher the temperature, the shorter the wavelength at which the maximum occurs. Fig. 4 shows one family of curves for a typical body. Spectral radiant emittance (in watts/cm$^2$) is plotted against wavelength (in micrometres) for a series of temperatures.

By differentiating Planck's formula with respect to $\lambda$, and finding the maximum, Wien's formula is derived:

$$\lambda_{max} = \frac{2898}{T} \; [\mu\text{m}]$$

This formula expresses mathematically the common observation that colours vary as the temperature of a thermal radiator increases. For a temperature close to 32°C (about 305K), which is close to the human skin temperature, $\lambda_{max}$ = 10 $\mu$m. This is the wavelength of the radiation of highest intensity that the skin emits, and it belongs to the far infra-red region.

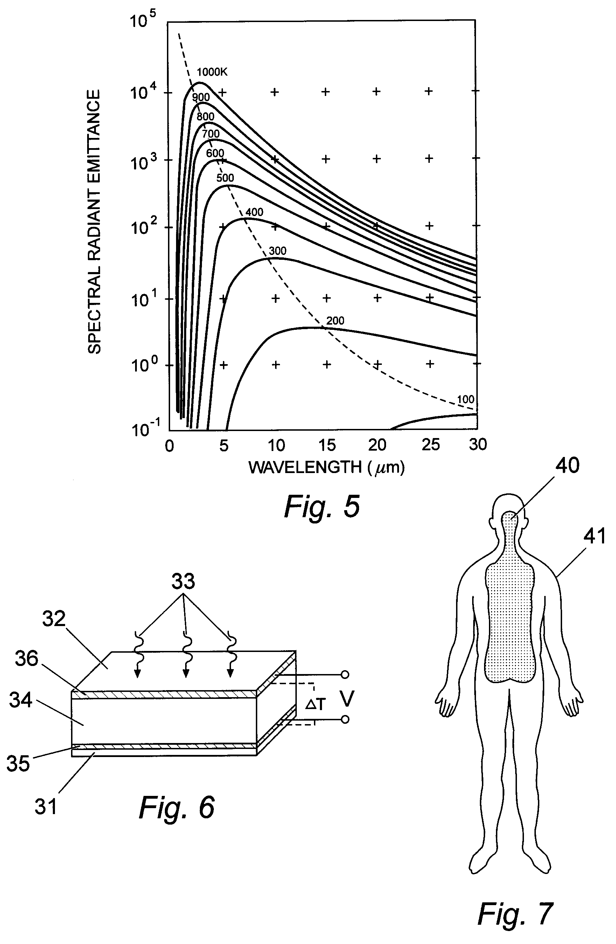

In Fig. 5 a semi-log plot of the Planckian curves is presented, and the part of the spectrum that corresponds with the radiation emitted by human skin appears (300 K). Spectral radiant emittance (in watts/cm$^2$) is plotted against wavelength (in micrometres) for a series of temperatures.

By integrating Planck's formula from $\lambda = 0$ to $\lambda = \infty$, the total radiant emittance ($W_b$) of a blackbody is obtained.

$$W_b = \sigma T^4 \; [\text{Watts/m}^2]$$

where $\sigma$ = Stefan-Boltzmann constant ($5.7 \times 10^{-8}$ W/m$^2$)

This is the Stefan-Boltzmann formula, which states that the total emissive power of a blackbody is proportional to the fourth power of its absolute temperature. Graphically, $W_b$ represents the area under the Planck curve for a particular temperature (see Figs. 4 and 5).

Infra-red (IR) detectors are available in many different forms, but only some of these meet the requirements for medical thermal imaging. These have a fast response time, are most sensitive at $\lambda$ between 1-20 $\mu$m, and (for some devices) have an ability to be incorporated into arrays of detectors. IR detectors are used to convert infrared energy into electrical signals, and can be either thermal detectors or photon detectors. Devices which detect infra-red radiation remotely range from simple point bolometers and thermoelectric detectors to the more sophisticated imaging systems such as scanners and pyroelectric vidicons. The method of the invention may make use of any of these devices, which are described in more detail below.

Pyroelectric detectors (see Fig. 6) are made from ferroelectric crystals which have an electrical polarisation strongly dependent on temperature.

A temperature difference (Delta T) is induced between the thermal insulation layers 31 and the absorbing black layer 32. This is proportional to the chopped incident radiation power 33 (and wavelength) absorbed by the black layer . A voltage signal V is then generated by the ferroelectric crystal 34 of pyroelectric material between the electrodes 35, 36, and this signal is also proportional to the temperature difference and therefore to the incident radiation wavelength.

Bolometers comprise thin metallic resistance elements onto which radiation is focused by an infra-red lens or mirror. Their resistance varies with temperature. Both devices operate using thermal detectors.

An electrical output is obtained from these devices by mechanically chopping the radiation falling on the crystal. Both devices are portable and can be used to give accurate temperature measurements by pointing them at a small area of skin, usually from close range.

Photoconductive detectors such as Indium Antimonide (InSb) or Mercury Cadmium Telluride (MCT) are photon detectors which operate on the principle that the material's crystal conductivity varies according to the number of incident infrared photons in its sensitive waveband. These detectors require cooling by liquid nitrogen which limits their mobility but by using a number of focusing and scanning mirrors they have considerable flexibility.

The Thermographic Imaging system used most successfully in the method of the invention is of the type described above (Agema Thermovision 880). The detector used is an MCT element (long wave version) covering 8-12 µm of the infra-red bank (Agema operating manual). In such a scanner electromagnetic energy radiating from the object being scanned is focused by an infra-red lens into a mirror. The mirror is oscillated by a dc motor. The optical output from the oscillating mirror is focused by three fixed mirrors onto a horizontal mirror polygon which rotates at 15000 rpm. Both the oscillator mirror and the horizontal mirror polygon are controlled by a microprocessor. The microprocessor provides horizontal and vertical trigger pulses to the Control Unit trigger circuit. The temperature resolution of the scanner is 0.05°C.

This scanner operates across the temperature range 15-40°C and the incoming signal from the detector is digitised. After that the thermographic image is displayed on a colour monitor, and the digitised data can be stored on computer discs in order to be processed later.

The Pyroelectric Vidicon tube is a newer development in thermographic imaging and is also suitable for use in the method of the invention. It operates in a similar manner to the optical vidicon camera with the exception that the target material in the tube is pyroelectric (ferroelectric crystals whose electrical polarisation alters with temperature).

There is an important requirement for all the infra-red imaging systems capable of precise temperature measurement. A thermal reference is required for calibration. This can either be used in the field of view (near to the material to be scanned) or incorporated into the optical system.

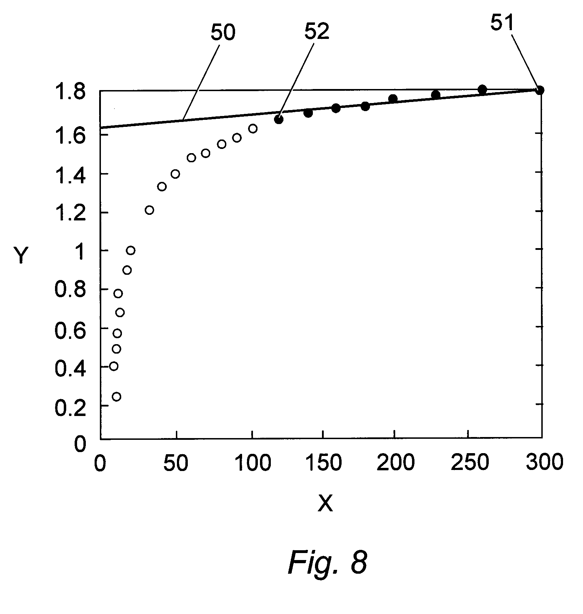

The method of the invention makes use of dynamic thermography, which is concerned with the thermographic imaging of the human body by detecting the infra-red radiation that is emitted by the skin. In an ambient temperature of 22°C a healthy person might have a skin temperature that ranges from 35°C over the sternum to 25°C over the feet. As discussed before, at these temperatures peak infra-red emission occurs around 10 µm wavelengths. Using a scanner that is sensitive in this region of radiation, a temperature map of the skin is acquired. Interpreting this information, an experienced physician can diagnose different sorts of abnormalities. A simple but useful concept when discussing internal body temperature is that of the centre "core" (see Fig. 7).

This "core" 40 includes the vital organs of the body 41 located in the trunk and head. Its mean temperature, for healthy subjects at rest, is around 37°C. The study of mechanisms by which the thermal energy is transferred from deeper tissues to the skin for subsequent exchange with the environment can indicate much about physiological mechanisms in health and disease.

Infrared thermography can be used to detect changes in the vascularity of the skin caused by a variety of internal disorders. Static thermography has been successfully applied in the thermographic assessments of joints, particularly for observing the response to treatment of conditions such as synovitis. Static thermography has been used for breast tumour diagnosis but failed in favour of mammography, since it pointed to many false positive diagnoses. Today static thermography is used for limb amputation level assessments, diagnoses of deep vein thromboses, and inflammations (e.g. back pains). However the simple and quick production of an image which indicates how temperature changes across an area of skin and which can be readily interpreted by medical practitioners or surgeons would be of great benefit, and would provide a further non-invasive diagnostic tool.

The following is an example of a *dynamic* thermographic procedure according to the invention.

- A cooling element (25°C) was placed on the subjects forearm for 15s so that the skin temperature decreased.
- Immediately after removal of the cooling block, the image capture program was activated when the skin site was held in a stable position.
- Thirty images were collected: 10 images at 1s intervals, 4 images at 3s, 8 images at 10s, 5 images at 20s, 3 images at 30 sec. The total time of acquisition was five minutes.
- The collected data were converted to ASCII format using a conversion program. During the conversion the option that produced images having the higher pixel resolution was selected (136 by 136).

The above procedure was carried out in two series of images, one recorded from normal skin and the other from inflamed skin, 48 hours after intradermal injection of tuberculin Purified Protein Derivative (PPD).

Coefficients of an exponential fit equation for the variation with time of the thermographic data were calculated using software decomposition techniques.

Fitting curves to multi-exponential data is a challenging problem encountered in many medical research areas fields such as magnetic resonance imaging (MRI) studies, pharmacokinetics, radioactive tracer techniques, blood flow studies and $H_2$ clearance studies. The solution of the present invention is applicable to these problems also.

According to the invention a multi-exponential curve decomposition technique was applied, in order to fit a curve to measured data. This data had been acquired during dynamic thermographic measurements of human skin as described above. The data was treated with a regression method called curve stripping, curve peeling or successive subtraction. In a graphic solution data was plotted on semi-log graph paper. A line was then drawn through the last points of the curve ("tail"), and its extrapolated values were subtracted from the first points of the curve ("front"). The process was repeated until there was no more data.

The approach can be illustrated graphically. Fig. 8 illustrates the first step in the curve stripping technique and shows one set of log-data. Measured data is plotted logarithmically on the Y axis against the varied parameter on the X axis. A line 50 is drawn from the last point 51 towards the first points. This line is the first fit-line and has a satisfactory correlation with the last 8 points 51 through to 52. This line gives the A component of the exponential equation.

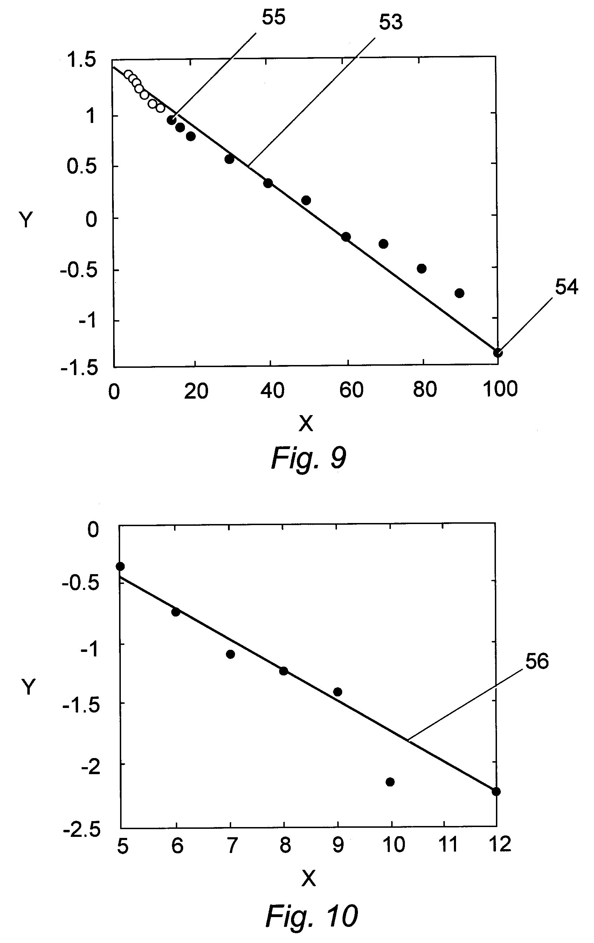

Fig. 9 illustrates the next step, the calculation of the B component of the exponential equation. After the calculation of the fitted values for the first line, the method continues subtracting these values from the original (experimental) ones. The residuals are the data set for the second fit-line, again plotted on a logarithmic Y axis against a linear X axis for the varied parameter. Another line 53 is drawn from the end 54 towards the front and this time has a satisfactory correlation with 11 data points 54 through to 55. This line gives the B component.

It should be noted that in the graphical method the selection of data points which either belong or do not belong to a fit-line is done by visual observation of the data set. The first 7 points that are left for the next fit-line seem to belong to a different data "family" from those data points which fit with this second fit-line. Although one line could be fitted to all the points of Fig. 9 it seems that this would not be right. This "visual" nature of the curve stripping method is discussed in more detail below.

The last step of this fitting procedure is shown in Fig. 10, which shows the calculation of the C component of the exponential equation using fit-line 56 in a similar way to that of the B component described above.

After the calculation of the 3 fit-lines (A, B and C components), their exponentials are derived and the multi-exponential fit equation is the summation of these exponentials.

Although the curve stripping technique is simple it gives satisfactory results for many different types of experimental data. It requires a minimal amount of computer processing capacity, so that there is no reason to use a method that requires access to a large computer, a lot of computing time and which produces, in the end, only a slightly better fit.

An advantage of the curve stripping technique is that it is a visual technique, requiring the user to trust one's eyes rather than numbers. Numerical calculation may be more accurate, but the experienced eye can "detect", "evaluate" and finally "decide" much more efficiently and correctly than any "sophisticated" mathematical procedure.

The visual quality of this method has been of major interest in the implementation of the software and much consideration was given into how best to include this feature in the software.

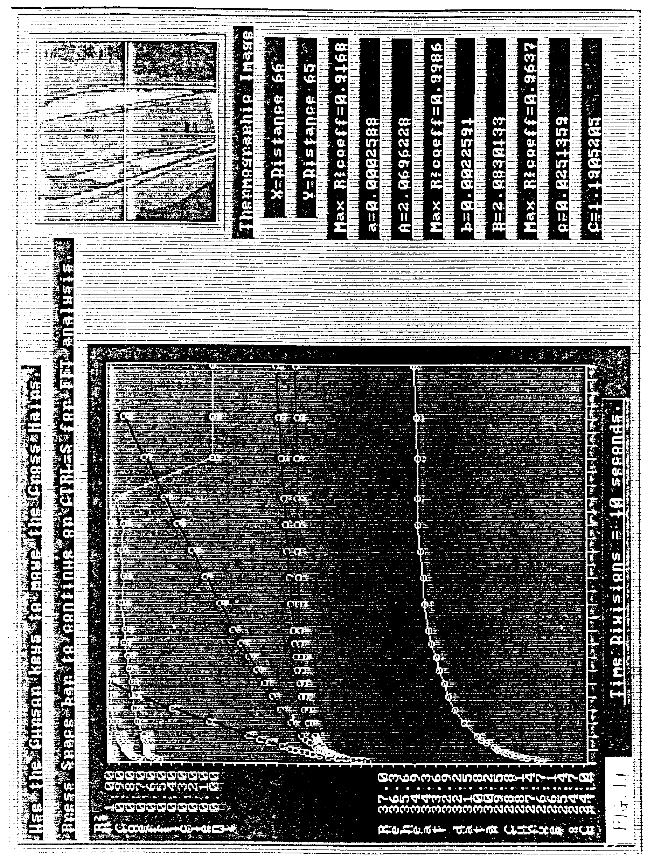

Fig. 11 shows a typical screen image obtained from the software showing the reheat data curve at a chosen location and the coefficients calculated by the software curve stripping method for the reheat data curve. The screen image shows the curves used to calculate the coefficients as well as the coefficients themselves. The three upper lines show the $R^2$ value (square of the correlation coefficient) for coefficients B, C and A respectively from the top. The reheat data curve is indicated by the lower line from 26°C to 35°C. The log-data curves are shown by the intermediate four lines.

In order to create the algorithms for reconstructing the parameters, various software was developed. This will now be described in more detail. The software programming languages QBASIC, Turbo C++ and TurboBASIC were used.

Two sets of data were processed using the software, one from normal skin thermographic images, and one from images of the tuberculin reaction. Six basic procedures were implemented by the software as follows:

- Conversion from AGEMA image file format to either a binary or ASCII file format.
- Creation of a Large Image File.
- Image cropping using a polygon mask.
- Creation of a Reheat Data File.
- Curve stripping or curve decomposition into exponential components.
- Parametric file image display.

Conversion from AGEMA image file format to either a binary or ASCII file format

The AGEMA thermographic camera produces its own particular image file format containing the temperature information (each pixel of the 136*136 matrix represents a temperature value) and calibration data. Programs CONVERT1 and CONVERT2 were used to convert the original AGEMA image files into ASCII and binary files respectively. The CONVERT2 program also produced an additional file that contains the paths and file names of the original AGEMA files and the converted output binary temperature files. This filename is automatically called by the output file name "FILENAME" which has been generated by the user and has a .VAR extension and is located in the same directory as the one specified by the user when entering the output filenames and directory details. The file also contains the timing information which is required for the curve stripping algorithm. The format for the FILENAME.VAR file is as follows.

NUMBER_OF_FILES
MINTEMP
MAXTEMP
"SOURCE_PATH_AND_FILENAME.001","TARGET_PATH_AND_FILENAME.001"
"SOURCE_PATH_AND_FILENAME.003","TARGET_PATH_AND_FILENAME.002"
Etc...

The NUMBER_OF_FILES number specifies the number of files that have been converted by the AGEMA conversion utility and the MINTEMP and MAXTEMP numbers specify the minimum and maximum temperature range for the output binary temperature data files specified by the TARGET_PATH_AND_FILENAME string. The timing information is held in the extension of the SOURCE_PATH_AND_FILENAME character string.

The binary temperature data has a resolution of 254 discrete temperature values from the minimum temperature (normally 24 degrees Celsius binary value 00000000 to 37 degrees Celsius binary value 11111110). The value 255 binary 11111111 is not used as this is required for the image cropping algorithm.

Creation of a Large Image File

The software developed for the analysis of the dynamic thermographic image processing is called PROJECT1.

The program PROJECT1 was developed as an easy to use program with a simple menu system allowing the user to analyse and display the thermographic images and to quickly produce the parametric image files. After the conversion of the original AGEMA files to the output binary temperature image files using the AGEMA conversion program CONVERT2 (which may be run from a main menu available to the user), a composite file called a Large Image File is created, called FILENAME.LIF.

The FILENAME.LIF file is a binary file which combines the binary image files sequentially to produce one large file which is easier to manipulate using a single file pointer instead of opening dozens of individual files. This improves the speed and efficiency of the dynamic thermographic image processing software and saves the program PROJECT1 opening dozens of individual binary image files. The creation of the Large Image File is achieved by opening a file called FILENAME.LIF in the same directory as the binary image files. Each binary image data file is then opened sequentially and the data is added to the output FILENAME.LIF file stream until there are no more binary image files. Therefore, if there are 30 binary image files consisting of 136x136 pixel matrices (18496 bytes) the FILENAME.LIF file that would be generated is 30x18496 bytes in length, i.e. 554881 bytes in length. (Note the extra byte marks the End of File or EOF). A single file now contains all the thermographic image information. The next step in the parametric image processing is to define a Region of Interest (ROI) and to generate and store the reheat temperature data sets for each pixel in a separate file. The program PROJECT1 also has an algorithm to deal with the problem of slight but noticeable movements of the experimental subject during the temperature data acquisition. In order to detect any movement the user must specify an option (F8-PIXEL TEMPERATURE DISPLAY/APPLY MOVEMENT DETECTION) before the creation of the Large Image File.

Image Cropping Using a Polygon Mask

The dynamic thermographic image processing method obtains more accurate information about blood perfusion in the skin than can be achieved using only a single static thermographic image. The only region of skin which is in a dynamic mode is the area of skin which has been subjected to the cold challenge (i.e. has had a cooling element applied thereto). The area of the cold challenge is easily observed in the first thermographic image by the distinctive rectangular cooled area of skin where the cooling element has been placed prior to the acquisition of the series of the thermographic images. The remaining static regions of the thermographic images are therefore excluded to provide a region of interest (ROI) by applying a polygon mask and setting the excluded temperature data values to 255. In practice this was achieved by using the QBASIC command POINT. When the user is prompted to create the data reheat file the program PROJECT1 scans the thermographic image polygon mask using the POINT command and discards any reheat temperature data sets where the value of the pixel which is being scanned is set to 255. Therefore pixels within the region specified by the polygon described by the user are allowed to be used in the creation of the reheat data file (FILENAME.RHD). The reheat data file is in an ASCII format which contains the timing data, information text header, and the pixel spatial location X,Y followed by the reheat temperature data set. The information text header consists of a number of text strings which allow the user to enter information about the experimental subject such as name, age, location of the cold challenge and address etc. This is done by selecting a first option (F12-CHANGE INTERNAL SETTINGS/EDIT TIMING SEQUENCE), then selecting a sub-option (4-EDIT SUBJECT DETAILS) and then entering the appropriate text at each prompt. This option should be carried out before the creation of a Reheat Data File or the text header information will be missing.

Creation of a Reheat Data File

The reheat data file is generated by the program PROJECT2 and contains the following information in the following ASCII format.

```
"PATH_AND_FILENAME_OF_PARAMETRIC_DATA_FILE"
"DATE_OF_FILENAME.RHD_CREATION"
"TIME_OF_FILENAME.RHD_CREATION"
"HEADER_TEXT_FIRST_LINE"
"HEADER_TEXT_SECOND_LINE"
"HEADER_TEXT_THIRD_LINE"
"HEADER_TEXT_FOURTH_LINE"
"HEADER_TEXT_FIFTH_LINE"
"HEADER_TEXT_SIXTH_LINE"
"HEADER_TEXT_SEVENTH_LINE"
NUMBER_OF_FILES
TIME_VALUE(1)
TIME_VALUE(2)
TIME_VALUE(3)
.....
TIME_VALUE(NUMBER_OF_FILE)
XLOCATION,YLOCATION,TEMPERATURE(1).....
TEMPERATURE(NUMBER_OF_FILES)..... etc
```

Curve Stripping or Curve Decomposition into Exponential Components

The next and most important step is to implement the algorithm to perform the multi-exponential curve decomposition using a modified regression analysis technique. The software is used to perform a multi-exponential curve fit in order to define three exponential component functions for each reheat temperature data set. Each reheat temperature data set corresponds to one individual pixel within the specified Region of Interest (ROI) defined by the user. The object of the software is to construct a number of new images using the parameters derived from the exponential decomposition (hence the term parametric imaging). The parameters are the coefficients **A,a,B,c,C,c** of the mathematical model:

$$Tr(t) = minval + Aexp^{-at} + Bexp^{-bt} + Cexp^{-ct}$$

where **Tr(t)** is the reheat temperature variable,
where **minval** is the first temperature value in the reheat temperature data set, and
where **A,a,B,c,C,c** are the coefficients of the 3 exponential components.

The process of generating the coefficients **A,a,B,c,C,c** is a difficult one and the following method assigns values to the six variables in order to generate a reasonable correlated "fit" between the experimental reheat temperature data set and the values which are obtained when the six coefficients are used to reconstruct the theoretical or "predicted" temperature values using the above mathematical model.

The program used to perform the multi-exponential curve decomposition is called CURVE3.

The Reheat Data File (FILENAME.RHD) contains all the information that is required for the program CURVE3 to operate. The program CURVE3 performs the following tasks:

The program reads the first 10 lines of the file which contain the following:

```
"PATH_AND_FILENAME_OF_PARAMETRIC_DATA_FILE"   'datafile$

"DATE_OF_FILENAME.RHD_CREATION"               'date1$



"TIME_OF_FILENAME.RHD_CREATION"               'time1$

"HEADER_TEXT_FIRST_LINE"                      'datatext$

"HEADER_TEXT_SECOND_LINE"                     'datatextname$

"HEADER_TEXT_THIRD_LINE"                      'datatextage$

"HEADER_TEXT_FOURTH_LINE"                     'datatextlocation$

"HEADER_TEXT_FIFTH_LINE"                      'datatextaddress1$

"HEADER_TEXT_SIXTH_LINE"                      'datatextaddress2$

"HEADER_TEXT_SEVENTH_LINE"                    'datatextaddress3$
```

The program then reads the following data:

```
NUMBER_OF_FILES                              'size%

TIME_VALUE(1)                                'timeval%(1)

TIME_VALUE(2)                                'timeval%(2)

TIME_VALUE(3)                                'timeval%(3)

......                                       '......

TIME_VALUE(NUMBER_OF_FILES)                  'timval%(size%)
```

The NUMBER_OF_FILES determines how many TIME-VALUE data values are to be read by the program. The TIME_VALUE data values are then read by the program and are then stored into the array timeval% (1-size%) array. The working arrays required for the curve decomposition are then dimensioned using the NUMBER_OF_FILES integer. This variable is called size% in the implemented program.

The output parametric data file FILENAME.DAT is opened in the directory specified in the PATH_AND_FILENAME_OF_PARAMETRIC_DATA_FILE (datafile$) string and the following header text output is written to the file:

```
"DATE_OF_FILENAME.RHD_CREATION"              'date1$

"TIME_OF_FILENAME.RHD_CREATION"              'time1$

"HEADER_TEXT_FIRST_LINE"                     'datatext$

"HEADER_TEXT_SECOND_LINE"                    'datatextname$

"HEADER_TEXT_THIRD_LINE"                     'datatextage$
```

```
"HEADER_TEXT_FOURTH_LINE"                        'datatextlocation$

"HEADER_TEXT_FIFTH_LINE"                         'datatextaddress1$

"HEADER_TEXT_SIXTH_LINE"                         'datatextaddress2$

"HEADER_TEXT_SEVENTH_LINE"                       'datatextaddress3$
```

The program then performs the following loop:

The program reads the **XLOCATION (xx%), YLOCATION (yy%)** and then the reheat temperature data set **TEMPERATURE(1)...TEMPERATURE(NUMBER_OF_FILES)** in to the array trhv (1..size%).

The program CURVE3 then carries out the subroutine called *curvestrip*. The subroutine *curvestrip* calculates the coefficients **A,a,B,c,C,c** and these parameters together with the XLOCATION and YLOCATION are then printed to the screen and written to the output data file FILENAME.DAT using the ASCII format.

**XLOCATION,YLOCATION,A,a,B,c,C,c**

The program then terminates when there are no more reheat temperature data sets, i.e. the end of the FILENAME.RHD file. The FILENAME.RHD and FILENAME.DAT files are then closed.

The FILENAME.DAT file is referred to as a parametric image file and the data contained in the file is used by the program RESULTS1 which will image each parameter to determine the nature of the spatial variations of each parametric image.

The subroutine *curvestrip* performs the following algorithm to produce the required coefficient parameters **A,a,B,c,C,c.**

The method of curve decomposition used in the software is very sensitive to any offsets which occur in the analysis of the reheat temperature data set. Therefore the minimum value is subtracted from each temperature value. The variable offsetemp is set to the first temperature data point trhv(1). Assuming that the proceeding temperature data points are incremental in value the variable offsetemp is subtracted from the reheat temperature data set held in the array trhv (2..size%) to produce the temporary variable diff. If the variable diff is negative, i.e. a temperature data point has a temperature which is less than the minimum temperature then the diff variable is set to zero. Since the natural logarithm of zero is undefined the diff variable is incremented by adding 1. Therefore any temperature less than the first temperature data point has the value of the natural logarithm of 1, i.e. zero. The natural logarithms of the reheat temperature data set are stored in the array lgrhv(1..size%) with the value 1lgrhv(1) always set to zero.

The curve decomposition begins its operation by working from the last data point and works backwards towards the first data point. In order to simplify the procedure it was decided to reverse the order of the two arrays timeval1%(1..size%) and lgrhv(1..size%) so that the algorithm can begin its operation by starting at the beginning of the arrays timeval%(1..size%) and lgrhv(1..size%) and therefore increment the array pointers in a positive manner. This is achieved using the subroutine *reverseorder*. The arrays called temporary(1..size%) and temporary%(1.size%) are used as temporary storage arrays to allow the order reversal in the arrays timeval(1..size%) and lgrhv(1..size%).

The evaluation of the first line can now take place.

The data that are to be processed are the natural logarithms of the original data. This means that a linear regression equation will regress the original data exponentially.

$$\text{Assume the regression equation:} \quad \mathbf{Y=at+b}$$

Calculating the exponential of this equation, the following equation is derived:

$$\exp^Y = \exp^{(at+b)} = \exp^{at} \times \exp^b = \mathbf{A} \times \exp^{at}$$

In order to evaluate which is the best fit for the logarithmic data the correlation coefficient values for the regression equations are calculated. The program starts calculating the correlation coefficient value beginning with the first five temperature data values of the inverted array, and proceeds towards the last point, adding one point at a time, applying the linear least squares fit correlation coefficient equation below:

$$R^2 = \frac{(\Sigma (T - T_m) \times (t - t_m))^2}{(\Sigma (T - T_m)^2 \times \Sigma (t - t_m)^2)}$$

Where:

$R^2 =$     The square of the correlation coefficient.
$T =$     The reheat temperature data value.
$T_m =$     The mean value of the reheat temperature data set.
$t =$     The corresponding time value.
$t_m =$     The mean value of the time values.

The calculation of the squares of the correlation coefficients is performed by the subroutine *computerrunningarrays* in the CURVE3 software. The coefficients of each of the linear regression equations are also calculated and are stored in the arrays called aval(1-size%) and bval(1-size%) with the squared correlation coefficients held in the array called regg(1-size%). Because the first four squared correlation coefficients are not computed the first four values of the array regg(1-size%) are set to zero. Typically these values should be near unity but it is justifiable to assume that the first linear regression line should have more than four data points to be significant. This formula is applicable to normally distributed data. Since the data under process are the natural logarithms of the initial data it is reasonable to assume that the distributions they follow is close to normal. The program uses these correlation values only for comparison between different linear regression equations to determine the most appropriate "fit". The algorithm is essentially trying to determine the best fit line by emulating the visual method which has been used previously with some success.

The variable rmax is initially set to the value of the squares regression coefficient array value regg(ty%) where ty%=5.

In order to find the maximum squared correlation coefficient the program then differentiates the array regg(1-size%) from the fifth regression coefficient value to the (size%-8)th regression coefficient value using a 3 point backwards difference technique, with the differentiated values being stored in the array called diffregg(ty%-(size%-1)).

The algorithm then searches for the last maximum squared correlation coefficient value by testing for the condition where the last positive to negative transition occurs in the array diffregg(ty%-(size%-1)). If the above condition occurs the array pointer ty1% is set to the diffregg(ty%-(size%-1)) array pointer less two (t%-2). The pointer ty1% specifies the data point number used to determine the value of the linear regression coefficients held in the arrays aval(ty1%) and bval(ty1%). The variable maxreg is set to the value in the array regg(ty1%). The next test condition is to determine whether the gradient of the linear regression line is either positive or negative. If the gradient, i.e. the value of the array aval(ty1%) is negative, then the curve decomposition algorithm will continue to search for the condition where the value of the array aval(ty1%) is positive and the value of the array pointer ty1% is set to the new value where this condition applies. If the value of array aval(ty1%) is positive then the algorithm has successfully found the most appropriate linear regression line, i.e. the line which fits the natural logarithmic temperature data with the maximum correlation as indicated by the squared correlation coefficient value.

If there are no transitions from positive to negative values within the array diffregg(ty%-(size%-8), i.e. there are no maxima values of the array regg%(ty%-(size%-1)) then the process of finding the largest squared regression coefficient is implemented by resetting the array pointer t% to ty% and then, by using a FOR/NEXT loop, the curve decomposition algorithm searches for the maximum squared correlation coefficient. Again if the value of array aval(ty1%) is negative the curve decomposition algorithm will search for the condition where the array aval(t%) is greater than zero and the value of the maximum squared correlation coefficient exists. The value of the pointer ty1% is set to the array pointer t% and the best regression line has the coefficients defined by aval(ty1%) and bval(ty1%).

After the algorithm has found the most appropriate linear regression equation coefficients which best "fit" the first line evaluation of the natural logarithmic temperature data points, the algorithm then subtracts the exponential component function **exp(A).(-at)** from the existing logarithmic temperature data. This subtraction is performed by the subroutine *subtractparameters*. The temporary variables tavl and tbvl are set to the values of the coefficients of the linear regression equation and then the subroutine *subtractparameters* is called. The "new" logarithmic data variable newlg is found by carrying out the following.

```
newlg = lgrhv(n%) - (EXP (tbvl)* -tavl* timeval%(n%))
```

The lgrhv(n%) array is then set to value of newlg, the temporary values tavl and tbvl are reset to zero and the first value of the array lgrhv(I-size-%) is set to zero ready for the next curve decomposition. The first curve strip or decomposition is now completed with the parameter **A** being the value of the array bval(ty1%) and the **a** parameter being the value of the array aval(ty1%).

The second regression fit is achieved using a similar process working on the new logarithmic curve data with the data range set from the data point number (ty1%+1) to (size%-1). Again the squared correlation coefficient monitoring is used by recalculating the new values for the arrays regg((ty1%+1)-(size%-1)), aval((ty1%+1)-(size%-1) and bval((ty1%+1)-(size%-1). By finding the most appropriate linear regression fit the array pointer ty2% is obtained and the coefficients of the linear regression line are returned and the new parameters **B** and **b** are again subtracted from the previous logarithmic data set to form the next set of logarithmic data. The third regression fit is achieved using the same process.

The same curve stripping algorithm was also implemented in the program PROJECT1 and the graphical representations of the reheat temperature data arrays, logarithmic data arrays and the squared correlation coefficients array can be displayed by the user. The computed parameters **A,a,B,c,C,c** together with the maximum squared correlations $R^2$ are also displayed. To display the graphs the user can use the option (F7-DISPLAY REHEAT CURVE GRAPH) in the main menu of the PROJECT1 software. By moving the cross-hairs over the first thermographic image the user can display the reheat temperature data et of the individual pixel by pressing the RETURN key. The parameters **A,a,B,c,C,c** and **C,c** were then used to reconstruct the reheat temperature curve using the following equation.

$$\texttt{Tr(t) = minval + exp(1) + (exp(A) x exp(-at))}$$
$$\texttt{- (exp(B) x exp(-bt)) - (exp(C) x exp(-ct))}$$

(Note the exponential component time constants are the same values used in the mathematical model but the scaling coefficients **A,B,C** used in the imaging are the logarithms of the scaling coefficients used in the mathematical model described earlier.)

When this temperature function is plotted on the display against the experimental data the differences between the experimental data and the calculated model data can be graphically seen by the user. The mean value of the differences (mean value of error) was calculated using the following. (The same time values were used in calculating the "predicted" temperature values.)

$$\texttt{mean\_value\_of\_error= } \frac{\texttt{(} \Sigma \texttt{ (y-x) )}}{\texttt{N}}$$

where, **x** = the "predicted" data, **y** = the experimental data and **N** = the number of data points.

The mean value of error was displayed to give an indication of the "fit" between the experimental data and the predicted data. When the predicted results were calculated and displayed the results were sometimes disappointing and gave a poor visual "fit" whilst other reheat temperature curves gave excellent results. The mean value of error results were actually a poor indicator of curve reconstruction "fit", giving very low values with a poor visual "fit" and in many cases giving high values of error for curves which fitted very well indeed if the "offset component" is removed. This happens when the deviation of the calculated "predicted" curve evolves in a parallel manner to the experimental data. Two perfectly parallel similar curves have a correlation coefficient of 1, but a poor absolute mean value of error (differences). Other errors also became apparent. The "predicted" curve when reconstructed showed an initial excellent "fit" at the beginning of the experimental curve only to deviate badly as the "predicted" curve progresses to the end time values. Because exponential functions rapidly change as they progress any error in the coefficients of the exponential component functions will quickly become apparent. The errors in the coefficients of the exponential component functions are therefore important and can easily arise if for instance a single temperature data point with a considerable error gives rise to a wrong set of exponential coefficients during the first curve decomposition when there are only a few temperature values being used to calculate the squared correlation coefficients. Further work may be needed to produce an adaptive technique to "lock in" the two curves using a feedback technique to obtain even better accuracy in determining the parametric values.

Parametric File Image Display

The final stage of the software is a program which displays the parameters produced during the curve decomposition. As noted before the six parameters **A,a,B,c** and **C,c** are the coefficients of the mathematic model:

$$Tr(t) = minval + Aexp^{-at} + Bexp^{-bt} + Cexp^{-ct}$$

with **minval** being the first temperature value of the reheat temperature curve. The mathematical model above was chosen because of the underlying theoretical model of the thermodynamics of blood perfusion within the dermis. The equation has two variables, that of temperature and time and represents the temperature variation of an individual pixel with respect to time. The aim of the software is to produce a visual representation within a spatial context of the regional variations of the individual parameters.

The program RESULTS1 performs this task. The RESULTS1 program is an easy to use program using a simple menu system allowing the user to display the computer parameters from the curve decomposition. The program RESULTS1 can be run by using the appropriate option (F11-RUN THE PARAMETRIC IMAGE POST PROCESSOR DISPLAY PROGRAM) within the main menu of the PROJECT1 program.

The user is prompted to enter the path and filename of the FILENAME.DAT parametric image file which was created during the curve decomposition of the reheat curve temperature data. The program RESULTS1 is referred to as a graphics post processor. A routine for the detection of outliers has been implemented. In order to scale the parameters correctly and to assign the appropriate colours to the different discrimination levels, a range of values for each parameter has to be determined and a statistical approach was taken. The program opens the FILENAME.DAT file then reads the text header information which is then displayed to the user. The program then begins to read each parameter in the parametric data file and calculates the mean value and standard deviation of each parameter. By using a multiple of the standard deviation plus or minus the mean value, a range of values for each parameter can be set. In this case plus or minus three standard deviations were used. Any parameter value outside this range is then considered to be an outlier and is discarded. The parameter value range together with the parameter mean value and standard deviation are then displayed to the user.

Therefore the following was implemented:

The mean value and standard deviation of each parameter were computed.
The range between the mean value of each parameter plus and minus three standard deviations was defined.
Every parameter value that was outside the computed range was considered to be an outlier.

The user can then display the parameters using the easy to use menu system which was implemented within the parametric imaging software. In order to visualise the regional parameter variations a three dimensional display algorithm has also been implemented allowing the user to display graphically the parameters in an isometric form. By pressing F10 the user can display the parameters in an isometric form and adjustments to the scaling and display angle can be easily changed. Also included is a low-pass filter algorithm to smooth out some of the "noise" from the images generated.

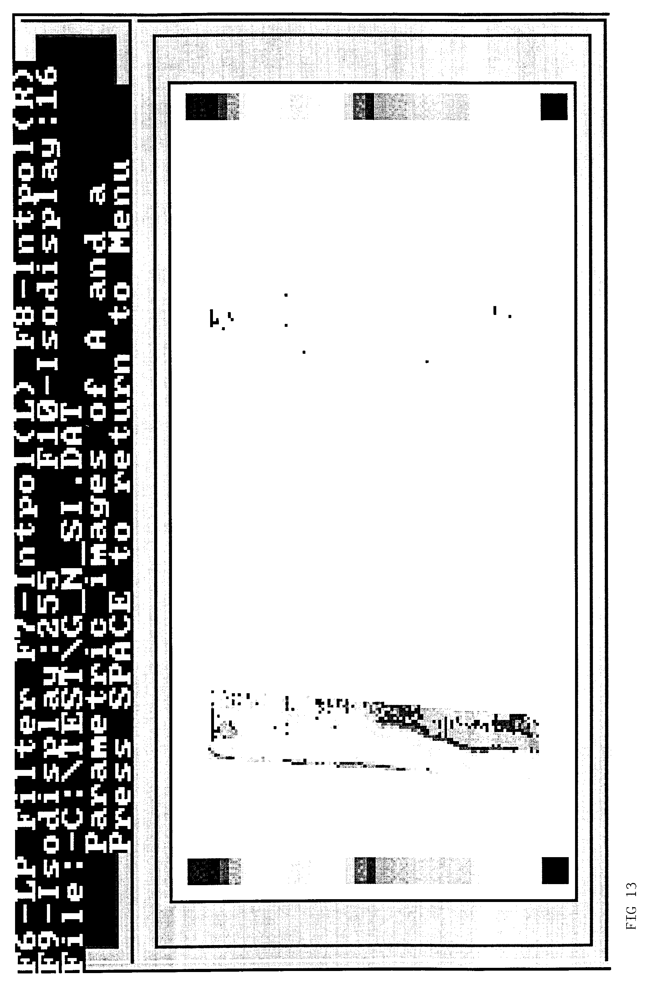

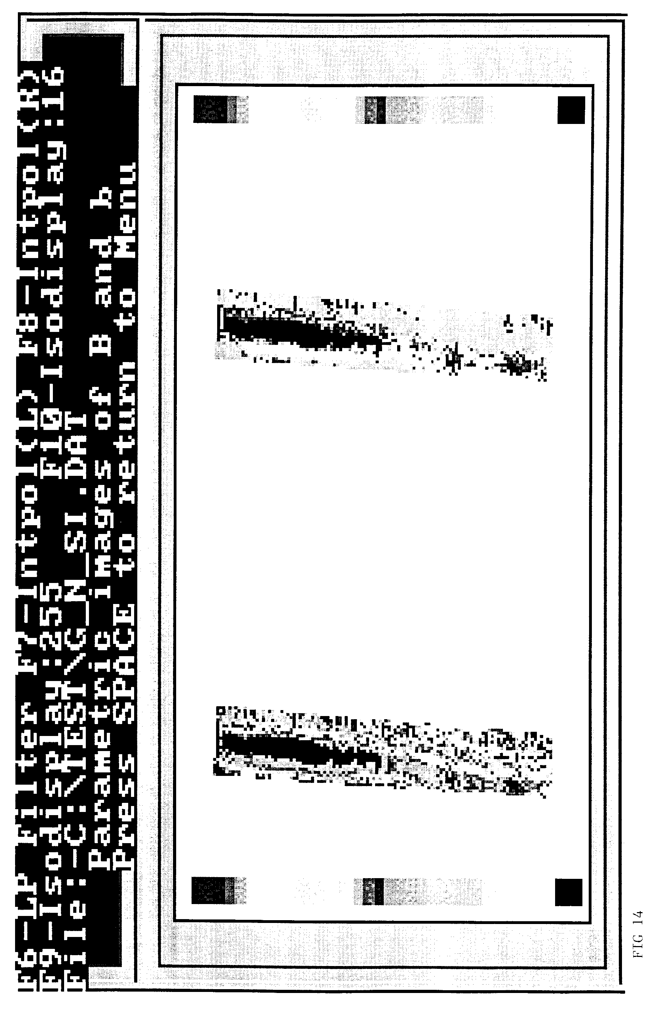

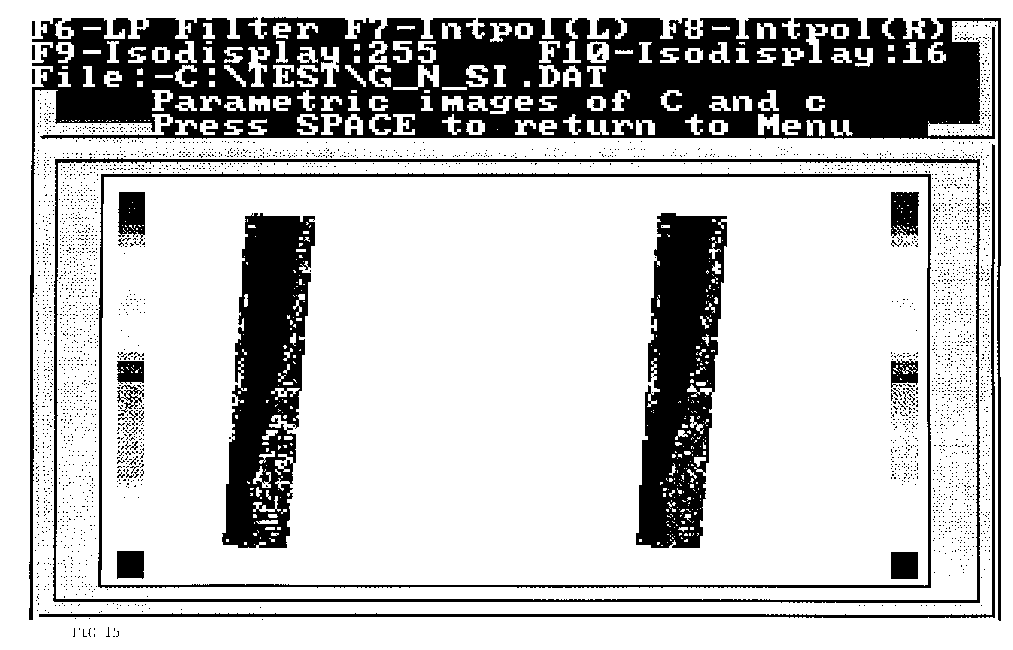

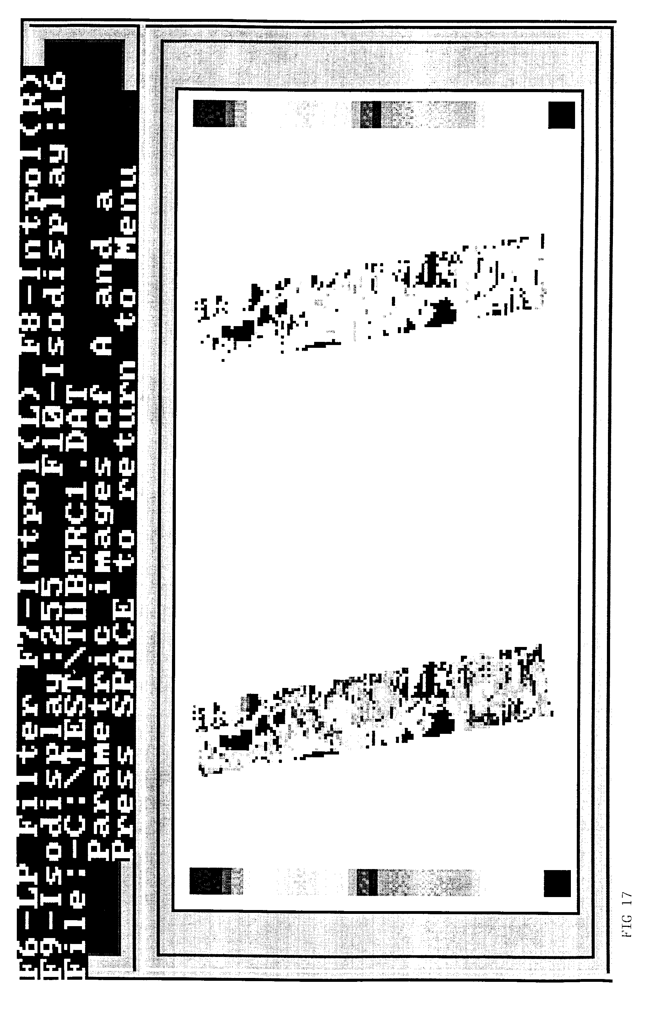

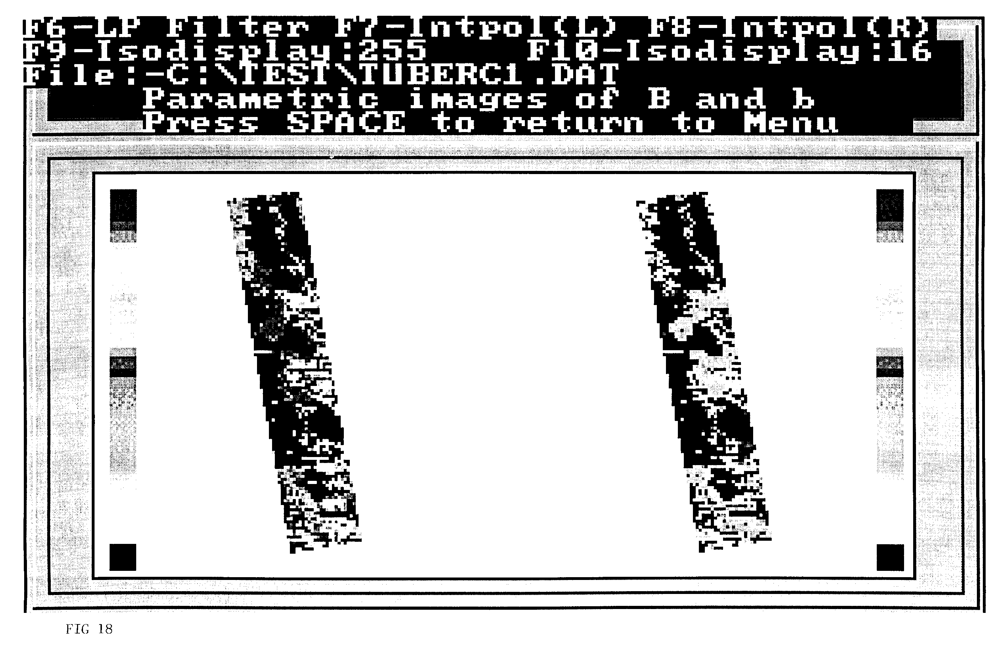

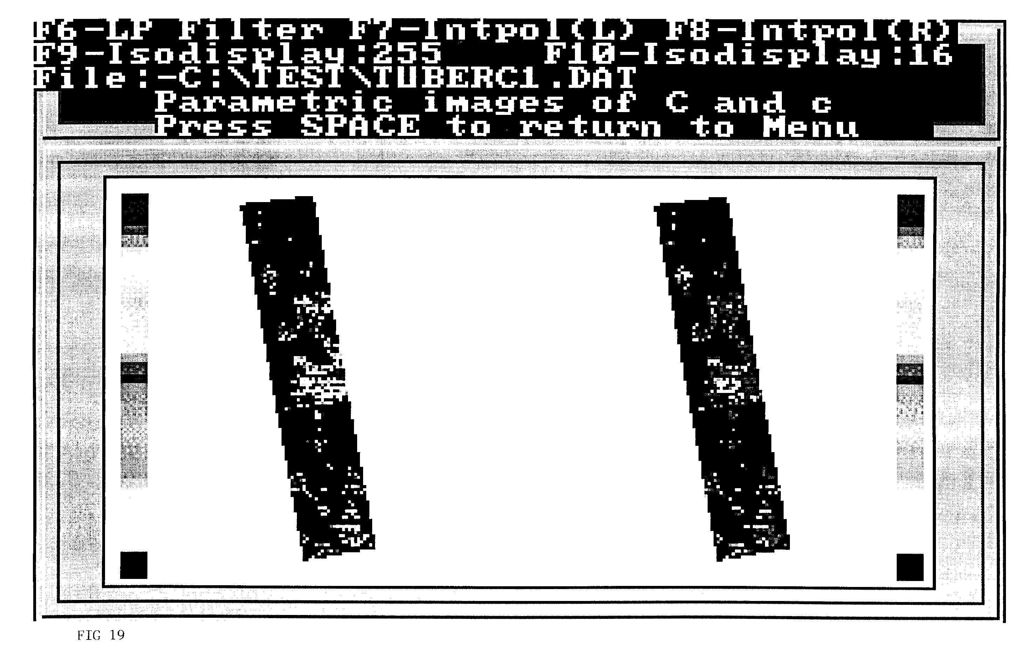

The images produced by the software can be seen in Figs. 13 to 15 (showing normal skin data) and Figs. 17 to 19 (showing tuberculin reaction skin data).

Dynamic Thermographic Image Processing Documentation File

The following is a summary of the files used in the thermographic image processing software method according to the invention and referred to in the description of the method, with a short description of each file.

**CONVERT1**  The AGEMA conversion utility source code which creates ASCII output data files written in TurboC++.
**CONVERT2**  The AGEMA conversion utility source code which creates binary output data files written in TurboC++.
**PROJECT1**  The thermographic image processing program written in QBASIC.
**RESULTS1**  The parametric image post processor program written in QBASIC.
**CURVE3**  The curve decomposition source program written in TurboBASIC.

Thus the invention provides a method of dynamic imaging of the blood perfusion rate in skin which, because of the speed at which the images can be created, is useful as a diagnostic tool for the medical profession.

The method is able to illustrate the changes in skin temperature rather than the skin temperature alone. These changes are directly related to blood perfusion rates in the deeper layers of the dermis having diameter >50μm.

The veterinary applications for infrared thermography are numerous also. Not only can it help veterinary surgeons make objective clinical diagnoses, but it can be used to monitor the progress of many different physiological disorders and gauge their response to various courses of treatment. It can immediately help with the identification of a whole range of problems, including: muscle disorders and secondary site of disease in lameness problems, conditions before symptoms show, eg tendons and joint disease, whether splints are active or inactive, back/spinal problems and identifying the difference between recent and long standing disorders, nerve injuries and circulatory disorders.

The curve stripping of multi-exponential curve decomposition is common to clinical studies. The underlying idea behind this modelling is the representation of a system with a finite number of component parts, called compartments or components. Each compartment contributes to the system according to its weighting factor, but has different origin, quality, and characteristics.

The temperature of the forearm's skin is the system under measurement. Previous studies indicated that skin temperature is a two-compartmental system. The method of the invention assumes that a third component could also fit the reheat curves.

Ultimately, 38.5% of the tuberculin reaction skin data (TRSD) and 55.9% of the normal skin data (NSD) revealed three components. Nevertheless, the two first components exist beyond any doubt, and the existence of a third component, is indicated for all the data. The physiological nature of the two components can be described as follows:

- a first component largely dependent on one or two compartments of blood flow
- a second component largely dependent on non-perfusion heat exchange mechanisms, including conduction

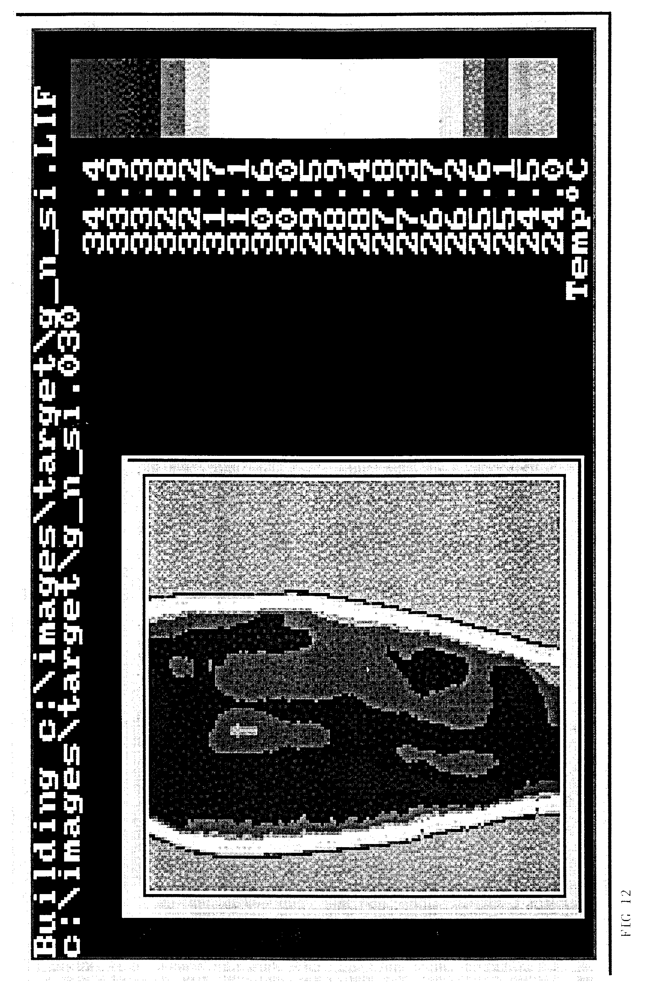

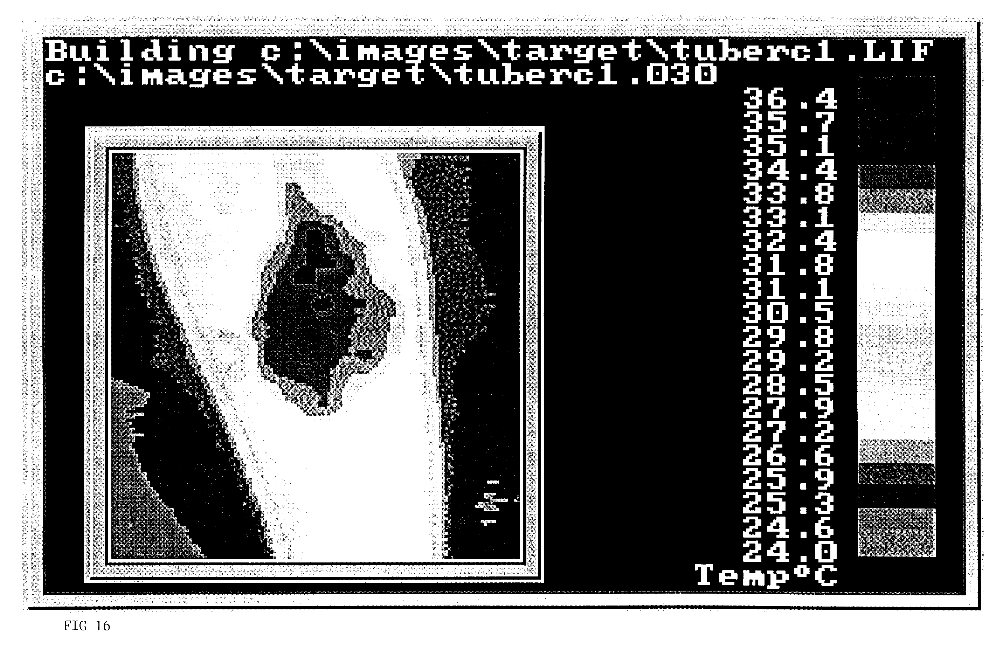

Figs. 13 to 15 and 17 to 19 illustrate the constructed coefficient images for the normal and the tuberculin reaction skin data respectively. One control thermographic image (acquired before the application of the dynamic thermographic procedure) is also displayed as Figs. 12 and 16 respectively at the start of each set of images. These two control images are both normalized from zero to one. The normalisation is done in each image's individual range (minimum-maximum values) and for this reason comparison between them in absolute temperature terms is not applicable.

On the other hand, comparisons between the two coefficient sets are more meaningful. The control images are displayed in order to be compared with the corresponding coefficient images.

For each component the "capital letter" image (A, B, C) is its weighting factor, and the other image (small letter a, b, c) can be interpreted as the one related to perfusion rates. The identification of which component corresponds to heat exchange due to blood perfusion and which to heat exchange due to other mechanisms is difficult at this stage on the basis of only two images.

After long consideration and observation of the images it was the first component that seems to be the component (largely) dependent on blood circulation (A, a). This approach illustrates the principle of higher flow (a) in small compartments (A) and vice versa.

An evidence that this evaluation is correct is the clear imaging of a vein that appeared in some parametric images and in the corresponding control images. The "A" value of the vein was low ("cold") and the a value was high ("hot"), leading to the physiological interpretation of high flow in small compartment.

The normal skin data Images did not show big differences compared to the control image. High flow in small compartments, and a heterogeneity level was obvious. A possible boundary effect was also displayed in A, a and b images, but not in the B image.

The tuberculin reaction skin data images were much more interesting. They illustrated clearly a high level of heterogeneity in red blood cell distribution (A) and velocities (a). There was considerably higher resolution of regional changes in blood circulation compared with the control image.

These images indicate the existence of very fine regulatory mechanisms controlling the blood flow in the deeper layers of the skin. The principle of high flow in small compartments is clear.

Dynamic thermography of the tuberculin reaction, followed by multi-exponential curve decomposition, indicates blood perfusion differences in a regional scale. Much better contrast in blood flow values is obtained compared to the static thermographic measurements.

The images illustrate the size of the compartments of blood flow together with the rate of flow through them.

Although particular software solutions are described above, it is to be understood that the invention is not to be limited to these particular software solutions, but is instead defined by the scope of the claims.

Similarly the apparatus of the invention should not be limited to those apparatus components specifically described above, but should include any infra-red detecting means which may be used to obtain periodically a set of thermographic data corresponding to skin temperature on an area of skin, including bolometers, thermoelectric detectors, scanners and pyroelectric vidicons; any data storage means which may be coupled to the detecting means and can store the thermographic data; any computational means which can be programmed to calculate the coefficients of an exponential fit equation, including separate PCs and dedicated microprocessor control units; and any image display means adapted to display the coefficients of the exponential fit equation in graphical form, including printers, monitors and TV screens.

These and other modifications and improvements can be incorporated without departing from the scope of the invention.

## Claims

1. A method of measuring blood perfusion in human or animal skin, comprising the steps of:

   (a) applying a cooling means to an area of skin;
   (b) removing the cooling means from the area of skin;
   (c) using an infra-red detector to obtain a first set of thermographic data $TD_1$ corresponding to skin temperature at a plurality of points on the area of skin at time $t_1$;
   (d) repeating step (c) at least once to obtain a total of n sets of thermographic data $TD_1$ to $TD_n$ corresponding to times $t_1$ to $t_n$, where n is at least 2;
   (e) calculating the coefficients of an exponential fit equation for the variation with time of the thermographic data $TD_1$ to $TD_n$ at each of the plurality of points; and
   (f) displaying the coefficients of the exponential fit equation in graphical form.

2. A method according to Claim 1, wherein the coefficients of the exponential fit equation are calculated using a curve stripping technique.

3. A method according to Claims 1 or 2, wherein n is at least 4, preferably at least 10.

4. A method according to any preceding Claim, wherein the coefficients of the exponential fit equation are displayed using colour mapping.

5. A method according to any preceding Claim, wherein the cooling means is a cold object which is placed on the skin.

6. A method according to any preceding Claim, wherein the method further includes the step of placing a reference object on the area of skin so that the thermographic data $TD_n$ is obtained at the same points on the area of skin at each successive repeat of step (c).

7. A method according to Claim 6, wherein the reference object is a reflective strip.

8. A method of processing and displaying data relating to blood perfusion in human or animal skin, comprising the steps of:

   (a) generating a series of n thermographic image matrices $IM_1$ to $IM_n$ corresponding to times $t_1$ to $t_n$, each thermographic image matrix comprising temperature data relating to a plurality of points on an area of skin;
   (b) creating a filter matrix corresponding to a region of interest selected from the area of skin;
   (c) applying the filter matrix to each thermographic image matrix $IM_1$ to $IM_n$ to obtain a cropped thermographic image matrix $IMCR_1$ to $IMCR_n$ having the value zero for points outside the region of interest;
   (d) constructing an array $AR_m$ for each point m in the region of interest, the array containing temperature data $T_m$ at that point corresponding to times $t_1$ to $t_n$;
   (e) for each array $AR_m$ performing a regression analysis to calculate the coefficients of the exponential components of the best fit equation

$$T_m = T_{min} + Ae^{at} + Be^{bt} + Ce^{ct}$$

   where A, a, B, b, C, c are coefficients and $T_{min}$ is the minimum temperature data in each array;

   (f) constructing a coefficient matrix ($Z_A$, $Z_a$ etc.) having as elements a parameter derived from the coefficients (A, a etc.) calculated in step (e) for each point m in the region of interest; and
   (g) displaying the coefficient matrix as an image.

9. A method according to Claim 8, wherein the parameter in step (f) is equal to one of the following:

(i) one of the coefficients (A, a etc.) calculated in step (e);

(ii) the sum of the coefficients A, B and C calculated in step (e), representing the total tissue volume;

(iii) the sum of the coefficient products (aA + bB + cC) divided by the sum of the coefficients (A + B + C), representing the total flow.

10. A method according to Claim 8 or 9, wherein the region of interest corresponds to an area of skin which has previously been cooled.

11. A method according to any one of Claims 8 to 10, wherein the coefficients of the exponential components are calculated using a curve stripping technique.

12. A method according to any one of Claims 8 to 11, wherein the minimum temperature data $T_{min}$ in each array is subtracted from each temperature data in the array before calculation of the coefficients.

13. A method according to any one of Claims 8 to 12, wherein the data corresponding to a first interval of time after cooling is discarded before calculating coefficients.

14. A method according to Claim 13, wherein the first interval of time is preferably less than 10 seconds, more preferably less than 5 seconds, most preferably between 3 and 4 seconds.

15. An apparatus for measuring blood perfusion in human or animal skin, comprising:

(a) an infra-red detecting means adapted to obtain periodically a set of thermographic data ($TD_1$ to $TD_n$) corresponding to skin temperature at a plurality of points on an area of skin;

(b) data storage means adapted to store each set of thermographic data ($TD_1$ to $TD_n$);

(c) computational means for calculating the coefficients of an exponential fit equation for the variation with time of the thermographic data ($TD_1$ to $TD_n$) at each of the plurality of points; and

(d) image display means adapted to display the coefficients of the exponential fit equation in graphical form.

Fig. 1

Fig. 2

Fig. 3

Fig. 4

*Fig. 5*

*Fig. 6*

*Fig. 7*

*Fig. 8*

Fig. 9

Fig. 10

FIG. 11

FIG 12

EP 0 885 587 A1

FIG 13

EP 0 885 587 A1

F6-LP Filter F7-Intpol(L) F8-Intpol(R)
F9-Isodisplay:255      F10-Isodisplay:16
File:-C:\TEST\G_N_SI.DAT
Parametric images of B and b
Press SPACE to return to Menu

FIG 14

F6-LP Filter F7-Intpol(L) F8-Intpol(R)
F9-Isodisplay:255      F10-Isodisplay:16
File:-C:\TEST\G_N_SI.DAT
Parametric images of C and c
Press SPACE to return to Menu

FIG 15

FIG 16

F6-LP Filter F7-Intpol(L) F8-Intpol(R)
F9-Isodisplay:255      F10-Isodisplay:16
File:-C:\TEST\TUBERC1.DAT
Parametric images of A and a
Press SPACE to return to Menu

FIG 17

F6-LP Filter F7-Intpol(L) F8-Intpol(R)
F9-Isodisplay:255      F10-Isodisplay:16
File:-C:\TEST\TUBERC1.DAT
    Parametric images of B and b
    Press SPACE to return to Menu

FIG 18

```
F6-LP Filter F7-Intpol(L) F8-Intpol(R)
F9-Isodisplay:255      F10-Isodisplay:16
File:-C:\TEST\TUBERC1.DAT
      Parametric images of C and c
      Press SPACE to return to Menu
```

FIG 19

**European Patent Office**

## EUROPEAN SEARCH REPORT

Application Number

EP 98 30 4752

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | GB 2 311 368 A (G. ROGERS ET AL.) 24 September 1997 * page 5, line 1 - page 9, line 27 * | 1,3,5, 10,14,15 | A61B5/00 |
| A | GB 2 298 489 A (G. ROGERS) 4 September 1996 * page 1, line 19 - page 9, line 21 * | 1,3,5,8, 14,15 | |
| A | US 5 056 525 A (F.F. HAFEZI) 15 October 1991 * abstract * * column 2, line 18 - column 3, line 5 * | 1,5,15 | |

| | | | TECHNICAL FIELDS SEARCHED (Int.Cl.6) |
|---|---|---|---|
| | | | A61B |

The present search report has been drawn up for all claims

| Place of search | Date of completion of the search | Examiner |
|---|---|---|
| THE HAGUE | 30 September 1998 | Rieb, K.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)